# EUROPÄISCHE PATENTSCHRIFT

(11) **EP 2 934 459 B1**
(45) Veröffentlichungstag und Bekanntmachung des Hinweises auf die Patenterteilung: **24.10.2018**
(21) Anmeldenummer: 13799075.0
(22) Anmeldetag: 04.12.2013
(51) Int. Cl.: A61K 8/36, A61K 8/365, A61Q 15/00, A61Q 17/00

(54) **VERBESSERTER SCHUTZ VOR KÖRPERGERUCH**
IMPROVED PROTECTION AGAINST BODY ODOR
PROTECTION AMÉLIORÉE CONTRE L'ODEUR CORPORELLE

(30) Priorität: 18.12.2012 DE 102012223532
(43) Veröffentlichungstag der Anmeldung: 28.10.2015
(73) Patentinhaber: Beiersdorf AG, 20253 Hamburg (DE)
(72) Erfinder: TRAUPE, Bernd, 24568 Kaltenkirchen (DE); BRINKMANN, Sina, 21698 Harsefeld (DE); URBAN, Michael, 22523 Hamburg (DE); SCHOLZ, Annika, 22844 Norderstedt (DE); KANINCK, Stefanie, 22359 Hamburg (DE); HALLMANN, Marita, 22417 Hamburg (DE); OELRICHS, Ilka, 25436 Tornesch (DE); ALDAG, Annabell, 21745 Hemmoor (DE); BÜRGER, Anette, 22529 Hamburg (DE)
(74) Vertreter: Hartmann, Jost
(86) Internationale Anmeldenummer: PCT/EP2013/075471
(87) Internationale Veröffentlichungsnummer: WO 2014/095366

(56) Entgegenhaltungen:
- EP-A1- 1 541 122
- EP-A1- 1 541 123
- EP-A2- 0 161 899
- WO-A1-97/15283
- GB-A- 2 163 652
- US-A- 4 518 582
- US-A1- 2007 275 064
- US-B1- 6 426 061
- SAXTON C A ET AL: "ANTIPLAQUE EFFECTS AND MODE OF ACTION OF A COMBINATION OF ZINC CITRATE AND A NONIONIC ANTIMICROBIAL AGENT", SCANDINAVIAN JOURNAL OF DENTAL RESEARCH, COPENHAGEN, DK, Bd. 96, Nr. 3, 1. Juni 1988 (1988-06-01), Seiten 212-217, XP001079620, ISSN: 0029-845X

## Beschreibung

Die Erfindung ist eine kosmetische oder dermatologische Zubereitung gemäß Anspruch 1, die eine gesteigerte desodorierende Wirksamkeit zeigen.

Als Schweiß wird ein von der Haut des Menschen über so genannte Schweißdrüsen abgesondertes wässriges Sekret bezeichnet. Körpergeruch entsteht, wenn der an sich geruchlose frische Schweiß durch Mikroorganismen wie z.B. Staphylokokken und Corynebakterien zersetzt wird.

Kosmetische Antitranspirantien oder Desodorantien/Deodorantien dienen dazu, Körpergeruch zu beseitigen bzw. deren Entstehung zu vermindern.

Die Wirksamkeit einer Antitranspirant (AT)-Zubereitung besteht einerseits in der schweißregulierenden Wirkung, anderseits in der Verhinderung von Schweiß- bzw. Körpergeruch.

Üblicherweise werden Aluminium-Salze als AT-Wirker in derartigen Systemen eingesetzt. Obwohl Aluminiumsalze neben der primären schweißregulierenden Wirkung auch desodorierende Eigenschaften aufweisen, besteht der stetige Wunsch nach verbesserter Wirksamkeit insbesondere in Hinblick auf die antimikrobielle bzw. desodorierende Wirkleistung.

EP 1541122 A1 offenbart desodorierende kosmetische Zusammensetzungen, die Zinkpidolat und Aluminiumchlorhydrat in aktivierter oder nicht aktivierter Form als antitranspiratorisches Aluminiumsalz umfassen.

Das Dokument EP 1541123 A1 offenbart ebenfalls desodorierende kosmetische Zusammensetzungen, die Zinkpidolat und Aluminiumchlorhydrat in aktivierter oder nicht aktivierter Form als antitranspiratorisches Aluminiumsalz umfassen.

Ebenso wird sowohl in EP 1541122 A1 als auch in EP 1541123 A1 die Verwendung der Kombination aus Zinkpidolat und Aluminiumchlorhydrat in aktivierter oder nicht aktivierter Form als desodorisierenden Wirkstoff in einer kosmetischen Zusammensetzung offenbart.

Zinksalze sind als Geruchsreduktionsmittel, als desodorierend, bekannt, wie beispielsweise in der US 4425321 beschrieben.

EP 768080 A1 offenbart desodorierende Zusammensetzungen umfassend wasserlösliche Zinksalze als Geruchsabsorber, insbesondere Zinkpidolat, Zinksulfat, Zinkchlorid, Zinklactat, Zinkgluconat.

WO 2006074359 A2 beschreibt die Verwendung von Zinksalzen in Kombination mit anderen antimikrobiell wirksamen Stoffen, wie Erythromycin.

US 5662937 offenbart langwirksame Deodorantzubereitungen umfassend Talk, Zinkoxid und Zitronensäure.

WO 2007076125 A2 beschreibt antimikrobiell wirksame Zubereitungen, die eine ZinkVerbindung enthalten, wie Zinkacetat, Zinkbutyrat, Zinkchlorid, Zinkcitrat, Zinkgluconat, Zinkgylcerat, Zinkformat, Zinklactat und weitere.

DE 102005032239 A1 beschreibt Zinkstearat als Trennmittel in einer Pudergrundlage.

DE 102008053034 A1 beschreibt die Verwendung von Hydroxyzimtsäuren und Pflanzenextrakten als Deo-Wirker. Hierbei werden generell adstringierende Salze des Aluminiums, Zirkoniums, Zinks oder Titans als schweißhemmende Wirkstoffe offenbart. Es werden in verschiedenen Beispielen Kombinationen von Aluminiumchlorohydrat (ACH (20 %)) mit Zinkgluconat, Zinkpyrrolidoncarbonsäure, Zinkcocoylethersulfat, Zinkglycinatmonohydrat oder Zinklactat beschrieben.

DE 102010055816 A1 offenbart die Kombination von ACH (20 %) und Zinklaktat (0,2 %) in Deo/AT-Sprays.

DE 60213209 T2 offenbart Pantothenatsalze in wasserfreien AT-Zubereitungen, wobei das Pantothenatsalz auch mit Zink gebildet werden kann. Die Pantothenatsalze sollen dabei die ACH-stabilere Alternative zu Panthenol darstellen.

DE 602004009495 T2 beschreibt Zinksalicylat in alkoholischen Deo-Sprays.

DE 602005000901 T2 beschreibt Zubereitungen zur Stabilisierung von W/O-Emulsionen, die mindestens ein desodorierendes Salz enthalten. Als desodorierendes Salz können alle bekannten AT- oder Deowirkstoffe gewählt werden.

In der US 6426061 B1 werden topische Zubereitungen zur Vermeidung von Schweißgeruch beschrieben, die relativ speziell auf Mechanismen der Geruchsentstehung eingehen und dabei Inhibitoren der Expression des Androgenrezeptors beanspruchen, wie Resveratrol, Epigallocatechin-3-gallate und Flufenamic Acid. Die Zubereitungen umfassen weiterhin zwingend den o.g. Inhibitor (a), sowie (b) einen Wirkstoff zur Inhibierung von Träger-Proteinen, wie Monensin, Tunicamycin, Amphmycin, Diumycin, Showdomycin, Tsushimycin, Amphortericine, Mycospocidin, Streptovirudin und D-Glucosamine, (c) Zink-Salze als anti-DHT (Dihyrotestosteron) Wirker, (d) einen AI-basierenden AT-Wirker und (e) einen zusätzlichen antimikrobiellen Wirkstoff, wie Chlorhexidindigluconat und Chlorhexidindiacetat.

US 7332154 B2 offenbart eine Kombination von Zinkgluconat und einem AI-basierenden AT-Wirker.

EP 1902754 A1 offenbart Kombinationen von wasserlöslichen Zinksalzen und Salicylsäure in Deo-Zubereitungen. Als wasserlösliche Zinksalze werden Zink PCA, Zinksulfat, Zinkchlorid, Zinklaktat, Zinkgluconat, Zinkphenolsulphonat und Zinksalicylat aufgeführt und in einer weiteren Ausführungsform in Kombination mit Al-Salzen beschrieben.

WO 2001089452 A2 beschreibt AT-haltigen W/O-Emulsionen mit geringem Wassergehalt in denen optional ein wasserlösliches Salz, wie Kochsalz aber auch Zinkcitrat und/oder Zinkglycinat genannt sind. Der Zusatz dient zur Stabilisierung von W/O-Emulsionen durch Elektrolyte.

Des Weiteren kennt der Fachmann die Dokumente GB 2163652 A, US 4518582 A, US 2007/0275064 A1 und WO 97/15283 A1, doch konnte keines dieser Dokumente einen Hinweis auf die vorliegende Erfindung geben.

Wünschenswert ist es antitranspirantwirksame Zubereitungen zur Verfügung zu stellen, die neben der AT-wirkung eine verbesserte Produktleistung hinsichtlich der Deo-Wirkung und dem Schutz vor Körpergeruch bieten.

Insbesondere ist es wünschenswert Zubereitungen zur Verfügung zu stellen, deren Anteil an antitranspirantwirksamen Verbindungen verringert werden kann ohne Einbußen hinsichtlich ihrer Wirksamkeit befürchten zu müssen. Ebenso ist es wünschenswert Zubereitungen zur Verfügung zu stellen, die länger deowirksam sind als aus dem Stand der Technik bekannte Zubereitungen und dies ohne Einbußen hinsichtlich der Hautverträglichkeit.

Die Erfindung ist eine kosmetische oder dermatologische Zubereitung gemäß Anspruch 1

Carbonsäuren sind organische Verbindungen, die eine oder mehrere Carboxygruppen (-COOH) tragen. Die Carbonsäuresalze werden Carboxylate und ihre Ester Carbonsäureester genannt.

Als erfindungsgemäße Zinksalze von Carbonsäuren wird das Zinksalz der Citronensäure gewählt.

Antitranspirantien oder Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

Die gewünschte Minimierung der Schweißsekretion kann durch verschiedene Mechanismen realisiert werden. Hierzu zählt traditionell der Einsatz von Adstringenzien, welche Eiweißfällungen und Gerinnungen hervorrufen und so eine Verengung oder Verschluss der Schweißdrüse bewirken.

Neuartige AT-Wirker basieren bspw. auf dem Prinzip der Anticholinergika, welche die Nervenreize, die zur Sekretionssteigerung der Schweißdrüsen führen, unterbrechen.

Ein weiteres Prinzip neuartiger AT-Wirker beruht auf der Beeinflussung von Membrantransportvorgängen in der Zelle. So hemmen spezifische Aquaporin-Inhibitoren die Proteine, die Kanäle in der Zellmembran bilden, um den Durchtritt von Wasser und weiteren Molekülen zu erleichtern. Auch lonenkanal-Hemmer bewirken eine Beeinflussung von Membrantransport- bzw. Osmose-Vorgängen. Ein weiterer neuartiger AT-Wirkmechanismus lässt sich durch die Verwendung kurzkettiger, vicinaler Diole realisieren, welcher möglicherweise auf deren osmotische Aktivität zurückzuführen ist.

So durch Anwendung eines Mittels kein Einfluss auf die Schweißsekretion ausgeübt, also keine antitranspirante Wirkung realisiert wird, liegt erfindungsgemäß kein Antitranspirant vor.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruches (Geruchsverbesserungsmittel)

Gängige Wirkmechanismen hierzu sind antimikro- bzw. antibakterielle Effekte, wie sie auch das nicht-kolloidale Silber zeigen, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Umsetzung zu wohlriechenden Stoffen umgesetzt werden.

Zubereitungen können neben den eigentlichen schweißhemmenden Wirkstoffen (AT-Wirker) zusätzlich auch Stoffe enthalten, die den mikrobiellen Abbau des Schweißes hemmen, wie z.B. Triclosan. Triclosan wirkt gegen Gram-positive und Gram-negative Keime sowie gegen Pilze und Hefen, woraus eine desodorierende, jedoch keine antitranspirante Wirkung resultiert, da aus der Beeinflussung der bakteriellen Hautflora keine Beeinflussung der Schweißsekretion abzuleiten ist.

Insbesondere bei Antitranspirantien auf Basis von Aluminiumsalzen kann im Prinzip auf den weiteren Zusatz von rein desodorierend wirkenden Substanzen verzichtet werden, da diese per se ein antimikrobielles Potential aufweisen. Des Weiteren wird durch das reduzierte Wasserangebot durch die verminderte Schweißsekretion auch das bakterielle Wachstum gehemmt. Davon unberührt bleibt die erfindungsgemäße Wirksamkeitsverbesserung durch Kombination mit ausgewählten Zinkverbindungen.

Als Antitranspirantwirkstoffe finden Aluminiumsalze Verwendung. Die früher eingesetzten, stark sauer wirkenden Salze Aluminiumsulfat oder -chlorid und die Agaricinsäure sind weitgehend durch Aluminiumhydroxychlorid und -alkoholate ersetzt worden. Die nachfolgende Auflistung vorteilhaft einzusetzender Antitranspirant-Wirker soll in keinster Weise einschränkend sein:
Aluminium-Salze:
   - Aluminium-Salze wie Aluminiumchlorid AlCl3, Aluminiumsulfat Al2(SO4)3
   - Aluminiumchloride der empirischen Summenformel [Al2(OH)mCln], wobei m+n=6
   - Aluminiumchlorhydrat [Al2(OH)5Cl] x H2O
      ▪ Standard Al-Komplexe: Locron P (Clariant), Micro-Dry (Reheis), ACH-331 (Summit), Aloxicoll PF 40 (Giulini).
      ∘ Aktivierte Al-Komplexe: Reach 501 (Reheis), AACH-324 (Summit), AACH-7171 (Summit), Aloxicoll P (Giulini), Aloxicoll SD100
   - Aluminiumsesquichlorhydrat [Al2(OH)4,5Cl1,5] x H2O
      ∘ Standard Al-Komplexe: Aluminum Sesquichlorohydrate (Reheis), AACH-308 (Summit)
      ∘ Aktivierte Al-Komplexe: Reach 301 (Reheis)
   - Aluminiumdichlorhydrat [Al2(OH)4Cl2] x H2O

Aluminium-Zirkonium-Salze:
- Aluminium/Zirkonium Trichlorhydrex Glycin [Al4Zr(OH)13Cl3] x H2O x Gly
   ∘ Standard Al/Zr-Komplexe: Rezal 33GP (Reheis), AZG-7164 (Summit), Zirkonal P3G (Giulini)
   ∘ Aktivierte Al/Zr-Komplexe: Reach AZZ 902 (Reheis), AAZG-7160 (Summit), Zirkonal AP3G (Giulini)
- Aluminium/Zirkonium Tetrachlorhydrex Glycin [Al4Zr(OH)12Cl4] x H2O x Gly
   ∘ Standard Al/Zr-Komplexe: Rezal 36G (Reheis), AZG-368 (Summit), Zirkonal L435G (Giulini)
   ∘ Aktivierte Al/Zr-Komplexe: Reach 908 (Reheis), AAZG-7167 (Summit), Zirkonal AP4G (Giulini)
- Aluminium/Zirkonium Pentachlorhydrex Glycin [Al8Zr(OH)23Cl5] x H2O x Gly
- Aluminium/Zirkonium Octachlorhydrex Glycin [Al8Zr(OH)20Cl8] x H2O x Gly Ebenso von Vorteil können aber auch Glycin-freie Aluminium/Zirkonium-Salze sein.

Aluminiumsalze können nicht nur als Pulver, sondern auch in gelöster "wässriger" Form eingesetzt werden.

Die Antitranspirant-Wirkstoffe aus den zuvor geschilderten Gruppen werden in den erfindungsgemäßen Formulierungen bevorzugt in einer Menge von 1 bis 30 Gew.%, bezogen auf die Gesamtmasse der Zubereitung, d.h. inklusive der ggf. vorhandenen Treibgase, eingesetzt.

Bei Einsatz von z.B. ca. 33,3 Gew.% ACH (Aluminium Chlorohydrate, 50 % aq.) in der Wirkstofflösung für ein Aerosol-Spray (ohne Treibgas) und einem Abfüllverhältnis von etwa 30 : 70 (Wirkstofflösung zu Treibgas) wird ein Anteil von etwa 5 Gew. % ACH im Endprodukt vorhanden sein.

Als Wirkstofflösung wird die Summe aller Bestandteile ohne das Treibgas bezeichnet.

AT-Mittel aus der Gruppe der Anticholinergika, wie beispielsweise 4-[(2-Cyclopentyl-2-hydroxyphenylacetyl)oxy]-1,1-dimethyl-piperidiniumsalze, insbesondere das 4-[(2-Cyclopentyl-2-hydroxyphenylacetyl)oxy]-1,1-dimethyl-piperidiniumbromid können zu einem Anteil von bevorzugt 0,05 bis 1,0 Gew.%, vorzugsweise 0,1%-0,7%, insbesondere 0,3%-0,5 Gew.%, bezogen auf die Gesamtmasse der Zubereitung zugesetzt werden.

Auch vicinale Diole und ähnliche Wirkstoffe aus der Gruppe der osmotisch aktiven Substanzen können als AT-Wirker den erfindungsgemäßen Zubereitungen zugesetzt werden.

Selbstverständlich ist es möglich weitere oder andere Antitranspirant Wirkstoffe und/oder Deodorantien als kosmetisch wirksamen Bestandteil zu zusetzen.

Überraschend und für den Fachmann nicht vorhersehbar wurde gefunden, dass die Kombination gemäß Anspruch 1 zu einem synergistischen Effekt hinsichtlich verbesserter Deowirkung und verbessertem Schutz vor Körpergeruch führt.

Die synergistische Verbesserung der Deowirkung zeigte sich in Untersuchungen zur Absterbekinetik von Testkeimen, die für den Körpergeruch verantwortlich sind.

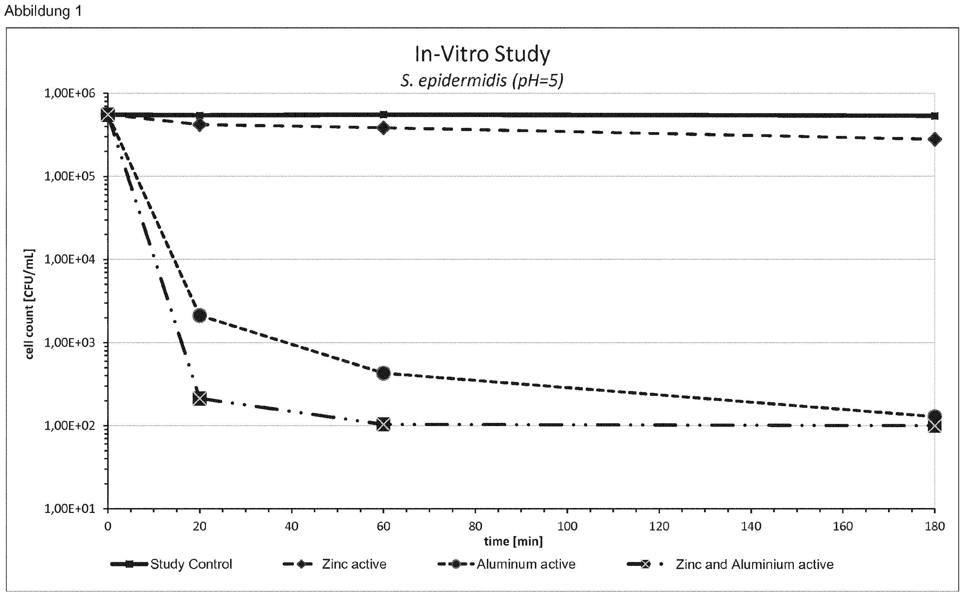

Abbildung 1 zeigt die Absterbekinetik des Testkeimes Staphylococcus epidermidis in einem in vitro Test-Design (Suspensionstest). Hierbei wurde die Reduzierung der Zellzahl (KBE) des Testkeimes nach Behandlung mit wässrigen Lösungen von Aluminiumchlorohydrat (ACH), Zinkcitrat und deren Kombinationen in geeigneter Konzentration über die Zeit bestimmt.

ACH alleine bewirkte erwartungsgemäß eine starke und schnell einsetzende Reduzierung des Testkeimes. Zinkcitrat hingegen zeigte alleine nur eine sehr schlechte Wirksamkeit gegen den Testkeim, insbesondere konnte kaum eine Sofortwirkung (Datenpunkte nach 20 und 60 Minuten) bestimmt werden. Überraschenderweise bewirkte jedoch die Kombination von ACH mit Zinkcitrat, dass die Absterbekinetik kurz- und mittelfristig deutlich verbessert werden konnte. Diese Wirksamkeitsverbesserung ist dabei nicht einfach additiv der beiden Substanzen, sondern, wie die Abbildung 1 zeigt, überproportional und damit synergistisch.

**Tabelle 1 zeigt die Testkeimzahl nach 20 Minuten**

| Probe | Zellzahl [KBE] t1 (20 Minuten), Median |
|---|---|
| ACH | 429,62 |
| ACH + Zinkcitrat | 103,50 |

Der den Tests zugrundeliegende Suspensionstest ist dem Fachmann als Standardmethode der DGHM (01.09.2001) bekannt.

Zinkcitrat ist als Bestandteil Zahnpflegeprodukten aufgrund der Antiplaquewirkung bekannt, wobei diese auf der antibakteriellen Wirksamkeit beruht. Da klassische AT-Mittel häufig bereits eine sehr gute antimikrobielle Wirksamkeit aufweisen war es erstaunlich, dass erfindungsgemäße Zinkcarboxylate und insbesondere Zinkcitrat eine weitere Wirksamkeitsverbesserung von AT-haltigen desodorierenden Produkten ermöglicht.

Vorteilhaft werden das oder die Zinksalze zu einem Anteil von 0,005 bis 5 Gew.%, insbesondere 0,01 bis 0,5 Gew.%, bezogen auf die Gesamtmasse der Zubereitung, eingesetzt

Vorteilhaft hinsichtlich der Wirksamkeitsverbesserung zeigte sich dabei ein bestimmtes Gewichtsverhältnis von AT-mittel zu Zinksalz im Bereich von 4000 zu 1 bis 2 zu 1.

So zeigte sich in Suspensionstests beispielsweise eine Kombination von 0,3 Gew.% ACH und 0,1 Gew.% Zinkcitrat vorteilhaft, also ein bevorzugtes Gewichtsverhältnis von 3 zu 1.

In einem erfindungsgemäß vorteilhaftem AT-Spray zeigte sich ein Gewichtsverhältnis von 35 Gew.% ACH zu 0,1 Gew.% Zinkcitrat, also ein Verhältnis von 350 zu 1, sowie in einem Roll-on Produkt ein Verhältnis von 10 Gew.% ACH zu 0,01 Gew.% Zinkcitrat, also 1000 zu 1, besonders vorteilhaft hinsichtlich der beschriebenen Wirksamkeitsverbesserungen.

Erfindungsgemäß wird auf den Zusatz von Wirkstoffen zur Inhibierung von Träger-Proteinen und/oder Inhibitoren der Expression des Androgenrezeptors, wie es im Stand der Technik beschrieben ist, verzichtet. Insbesondere ist kein Resveratrol, Epigallocatechin-3-gallat, Flufenamicsäure, Monensin, Tunicamycin, Amphmycin, Diumycin, Showdomycin, Tsushimycin, Amphortericine, Mycospocidin, Streptovirudin und/oder D-Glucosamine in den erfindungsgemäßen Zubereitungen enthalten.

Erfindungsgemäß ist auch die Verwendung gemäß Anspruch 7.

Die desodorierende Wirkung umfasst insbesondere den Schutz vor Schlechtgeruch. Dieser kann erfindungsgemäß durch eine erhöhte Reduzierung von Bakterien bzw. dessen Wachstum erreicht werden.

Zusätzlich zu den beschriebenen Verbesserungen der antimikrobiellen Wirksamkeit, die im in vitro Versuch für den Zeitraum bis zu 3 Stunden dargelegt wurde, konnte für die erfindungsgemäßen Produkte auch eine sehr gute in vivo Langzeitwirksamkeit (>24h, insbesondere 48h) gegen Körpergeruch nachgewiesen werden.

Die Zinksalze der Carbonsäuren lassen sich demnach in Kombination mit ein oder mehreren antitranspirant wirksamen Verbindungen in kosmetischen oder dermatologischen Zubereitungen zur Verminderung oder Verhinderung des Körpergeruches bis zu 48 Stunden nach topischer Applikation der Zubereitung verwenden, wobei als Zinksalz Zinkcitrat gewählt wird, als antitranspirant wirksame Verbindungen Aluminiumsalze gewählt werden und in den Zubereitungen keine Wirkstoffe zur Inhibierung von Träger-Proteinen und/oder Inhibitoren der Expression des Androgenrezeptors enthalten sind, insbesondere kein Resveratrol, Epigallocatechin-3-gallat, Flufenamicsäure, Monensin, Tunicamycin, Amphmycin, Diumycin, Showdomycin, Tsushimycin, Amphortericine, Mycospocidin, Streptovirudin und/oder D-Glucosamine.

Die kosmetischen oder dermatologischen Zubereitungen gemäß der Erfindung können ferner kosmetische Hilfsstoffe und Wirkstoffe enthalten, wie sie üblicherweise in solchen Zubereitungen verwendet werden, z. B. Wirkstoffe, Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen oder andere übliche Bestandteile einer kosmetischen oder dermatologischen Formulierung wie Polyole, Polymere, Schaumstabilisatoren, organische Lösungsmittel oder Silikonderivate, sofern der Zusatz nicht ausgeschlossen ist und die geforderten Eigenschaften hinsichtlich der AT- und Deowirksamkeit, Stabilität und Hautfreundlichkeit nicht beeinträchtigt.

Die Zubereitungen können als Spray, Roll-on, Stick oder Gel formuliert werden. Erfindungsgemäße Emulsionszubereitungen sind bevorzugt Wasser in Öl Emulsionen. Mikroemulsionen sind vorteilhaft ausgeschlossen.

Die nachfolgenden Beispiele veranschaulichen erfindungsgemäße Zubereitungen. Die Zahlenangaben sind Gewichtsanteile bezogen auf die Gesamtasse der Zubereitung.

### Beispiele

| AT-Suspensions-Spray | 1 | 2 | 3 | 4 |
|---|---|---|---|---|
| | % (w/w) | % (w/w) | % (w/w) | % (w/w) |
| Octyldodecanol | 0,80 | 1,00 | 0,90 | 0,8 |
| Tocopheryl Acetate | 0,06 | 0,08 | 0,07 | 0,1 |
| Dimethicone | 2,34 | 3,20 | 3,10 | 3,0 |
| Persea Gratissima Oil | 0,10 | 0,15 | 0,10 | 0,5 |
| Disteardimonium Hectorite | 4,00 | 3,80 | 3,90 | 3,5 |
| Propylene Carbonate | 0,00 | 0,00 | 0,00 | 0,9 |
| Isopropyl Palmitate | 0,00 | 0,00 | 0,00 | 10,0 |
| Cyclomethicone + Dimethiconol | 0,00 | 0,00 | 0,00 | 2,3 |
| Cyclomethicone | 49,00 | 51,47 | 48,50 | 32,89 |
| Aluminum Sesquichlorohydrate | 0,00 | 2,00 | 0,00 | 5,0 |
| Aluminum Chlorohydrate | 35,00 | 33,00 | 37,00 | 35 |
| Zinc Citrate | 0,70 | 0,30 | 0,10 | 0,01 |
| Parfum | 5,33 | 5,00 | 5,10 | 6,0 |
| Sodium Starch Octenylsuccinate + Mannitol | 2,67 | 0,00 | 1,23 | 0,00 |

Mit Treibgas vorzugsweise Butane/Isobutane/Propane und Abfüllverhältnis 5:95 bis 30:70, vorzugsweise 10:90 bis 20:80, vorzugsweise 15:85.

| AT W/O-Spray | 5 | 6 | 7 | |
|---|---|---|---|---|
| INCI | % (w/w) | % (w/w) | % (w/w) | % (w/w) |
| Aqua | 47,5 | 47,0 | 45,25 | 30 |
| Cyclomethicone | 11,75 | 11,75 | 11,75 | |
| Aluminum Chlorohydrate (50 % aq) | 20 | 20 | 20 | |
| Aluminum Sesquichlorohydrate (50 % aq.) | 0,00 | 0,00 | 2,0 | |
| C12-15 Alkyl Benzoate | 5 | 5 | 5 | |
| Octyldodecanol | 5 | 5 | 5 | |
| Dicaprylyl Ether | 4 | 4 | 4 | |
| Parfum | 3,3 | 3,3 | 3,3 | |
| Cetyl PEG/PPG-10/1 Dimethicone | 1 | 1,5 | 1,5 | |
| Sorbitan Trioleate | 1,5 | 1,5 | 1,5 | |
| Phenoxyethanol | 0,5 | 0,5 | 0,5 | |
| Butyloctanoic Acid | 0,25 | 0,25 | 0,00 | |
| Zinc Citrate | 0,1 | 0,1 | 0,1 | |
| Persea Gratissima Oil | 0,1 | 0,1 | 0,1 | |
| Butane, Isobutane, Propane | | | | 70 |

| AT-Sticks | 8 | 9 | 10 | 11 |
|---|---|---|---|---|
| | % (w/w) | % (w/w) | % (w/w) | % (w/w) |
| Octyldodecanol | 0,10 | 0,15 | 0,10 | 0,10 |
| Glyceryl Stearate SE | 0,60 | 0,60 | 0,65 | 1,00 |
| Persea Gratissima Oil | 0,05 | 0,07 | 0,05 | 0,10 |
| Hydrogenated Castor Oil | 1,50 | 1,50 | 1,60 | 1,50 |
| Talc | 4,00 | 2,50 | 3,65 | 2,00 |
| PPG-14 Butyl Ether | 15,00 | 14,43 | 0,00 | 20,00 |
| Caprylic/ Capric Triglyceride | 0,00 | 0,00 | 14,50 | 10,00 |
| Paraffinum Liquidum | 0,00 | 0,00 | 0,00 | 7,50 |
| Cyclomethicone | 41,25 | 38,10 | 37,94 | 15,70 |
| Aluminium Chlorohydrate | 0,00 | 20,00 | 0,00 | 0,00 |
| Aluminum Sesquichlorohydrate | 0,00 | 0,00 | 0,00 | 1,00 |
| Aluminum Zirconium Tetrachlorohydrex GLY | 16,00 | 0,00 | 20,00 | 19,00 |
| Stearyl Alcohol | 20,00 | 21,1 | 20,50 | 21,00 |
| Parfum | 1,00 | 0,9 | 1,00 | 1,00 |
| Zinc Citrate | 0,50 | 0,65 | 0,01 | 0,10 |

| AT Roll-on | 12 | 13 | 14 | 15 |
|---|---|---|---|---|
| | % (w/w) | % (w/w) | % (w/w) | % (w/w) |
| Aqua | 60,3 | 63,85 | 70,89 | 32,10 |
| Alcohol | 0,00 | 0,00 | 0,00 | 30,00 |
| Aluminum Chlorohydrate (50 % aq.) | 28,00 | 17,50 | 20,00 | 28,00 |
| PPG-15 Stearyl Ether | 3,00 | 0,00 | 3,00 | 0,00 |
| Octyldodecanol | 0,00 | 3,00 | 0,00 | 0,25 |
| Steareth-2 | 2,50 | 0,00 | 2,50 | 0,00 |
| Glyceryl Isostearate | 0,00 | 2,00 | 0,00 | 0,00 |
| PEG-40 Hydrogenated Castor Oil | 0,00 | 0,00 | 0,00 | 2,00 |
| Dicaprylyl Carbonate | 0,00 | 3,00 | 0,00 | 0,00 |
| Aluminum Sesquichlorohydrate (50 % | 2,00 | 2,50 | 0,00 | 2,00 |
| Trisodium EDTA (20 % aq.) | 1,50 | 0,00 | 1,50 | 0,00 |
| Hydroxyethylcellulose | 0,00 | 0,00 | 0,00 | 0,35 |
| PEG-150 Distearat | 0,00 | 0,80 | 0,00 | 0,00 |
| Isosteareth-20 | 0,00 | 4,00 | 0,00 | 0,00 |
| Steareth-21 | 1,50 | 0,00 | 1,00 | 0,00 |
| Parfum | 1,00 | 1,10 | 1,00 | 1,00 |
| PEG-8 | 0,00 | 0,00 | 0,00 | 3,00 |
| Glycerin | 0,00 | 2,10 | 0,00 | 1,00 |
| Persea Gratissima Oil | 0,10 | 0,10 | 0,10 | 0,00 |
| Zinc Citrate | 0,10 | 0,05 | 0,01 | 0,30 |

## Patentansprüche

1. Kosmetische oder dermatologische Zubereitung umfassend ein oder mehrere Zinksalze von Carbonsäuren und ein oder mehrere antitranspirant wirksame Verbindungen, die sich von den Zinksalzen unterscheiden **dadurch gekennzeichnet, dass** als Zinksalz Zinkcitrat gewählt wird, als antitranspirant wirksame Verbindungen Aluminiumsalze gewählt werden und in den Zubereitungen keine Wirkstoffe zur Inhibierung von Träger-Proteinen und/oder Inhibitoren der Expression des Androgenrezeptors enthalten sind, insbesondere kein Resveratrol, Epigallocatechin-3-gallat, Flufenamicsäure, Monensin, Tunicamycin, Amphmycin, Diumycin, Showdomycin, Tsushimycin, Amphortericine, Mycospocidin, Streptovirudin und/oder D-Glucosamine.

2. Zubereitung nach einem der vorstehenden Ansprüche **dadurch gekennzeichnet, dass** als antitranspirant wirksame Verbindung Aluminiumchlorohydrate gewählt werden.

3. Zubereitung nach einem der vorstehenden Ansprüche **dadurch gekennzeichnet, dass** der Gewichtsanteil an Zinksalzen im Bereich von 0,001 bis 5 Gew.%, insbesondere 0,01 bis 0,5 Gew.%, bezogen auf die Gesamtmasse der Zubereitung, gewählt wird.

4. Zubereitung nach einem der vorstehenden Ansprüche **dadurch gekennzeichnet, dass** der Gewichtsanteil an antimikrobiell und/oder antitranspirant wirksamen Verbindungen im Bereich von 1 bis 30 Gew.%, bezogen auf die Gesamtmasse der Zubereitung, gewählt wird.

5. Zubereitung nach einem der vorstehenden Ansprüche **dadurch gekennzeichnet, dass** das Gewichtsverhältnis zwischen antimikrobiell und/oder antitranspirant wirksamen Verbindungen und Zinksalzen im Bereich von 4000 zu 1 bis 2 zu 1 gewählt wird.

6. Zubereitung nach einem der vorstehenden Ansprüche **dadurch gekennzeichnet, dass** die Zubereitung keine Mikroemulsion ist.

7. Verwendung von Zinksalzen der Carbonsäuren in Kombination mit ein oder mehreren antitranspirant wirksamen Verbindungen in kosmetischen oder dermatologischen Zubereitungen zur Steigerung der desodorierenden Wirkung dieser Zubereitungen nach topischer Applikation **dadurch gekennzeichnet, dass** als Zinksalz Zinkcitrat gewählt wird, als antitranspirant wirksame Verbindungen Aluminiumsalze gewählt werden und in den Zubereitungen keine Wirkstoffe zur Inhibierung von Träger-Proteinen und/oder Inhibitoren der Expression des Androgenrezeptors enthalten sind, insbesondere kein Resveratrol, Epigallocatechin-3-gallat, Flufenamicsäure, Monensin, Tunicamycin, Amphmycin, Diumycin, Showdomycin, Tsushimycin, Amphortericine, Mycospocidin, Streptovirudin und/oder D-Glucosamine.

8. Verwendung von Zinksalzen der Carbonsäuren in Kombination mit ein oder mehreren antitranspirant wirksamen Verbindungen in kosmetischen oder dermatologischen Zubereitungen zur Verminderung oder Verhinderung des Körpergeruches bis zu 48 Stunden nach topischer Applikation der Zubereitung **dadurch gekennzeichnet, dass** als Zinksalz Zinkcitrat gewählt wird, als antitranspirant wirksame Verbindungen Aluminiumsalze gewählt werden und in den Zubereitungen keine Wirkstoffe zur Inhibierung von Träger-Proteinen und/oder Inhibitoren der Expression des Androgenrezeptors enthalten sind, insbesondere kein Resveratrol, Epigallocatechin-3-gallat, Flufenamicsäure, Monensin, Tunicamycin, Amphmycin, Diumycin, Showdomycin, Tsushimycin, Amphortericine, Mycospocidin, Streptovirudin und/oder D-Glucosamine.

## Claims

1. Cosmetic or dermatological preparation comprising one or more zinc salts of carboxylic acids and one or more antiperspirant compounds which differ from the zinc salts, **characterized in that** the zinc salt selected is zinc citrate, the antiperspirant compounds selected are aluminium salts and the preparations contain no active ingredients for inhibiting carrier proteins and/or inhibitors of the expression of the androgen receptor, more particularly no resveratrol, epigallocatechin-3-gallate, flufenamic acid, monensin, tunicamycin, amphmycin, diumycin, showdomycin, tsushimycin, amphotericins, mycospocidin, streptovirudin and/or D-glucosamines.

2. Preparation according to any of the preceding claims, **characterized in that** the antiperspirant compound selected is aluminium chlorohydrates.

3. Preparation according to either of the preceding claims, **characterized in that** the proportion by weight of zinc salts is selected within the range from 0.001 to 5% by weight, more particularly 0.01 to 0.5% by weight, based on the total mass of the preparation.

4. Preparation according to any of the preceding claims, **characterized in that** the proportion by weight of antimicrobial and/or antiperspirant compounds is selected within the range from 1 to 30% by weight, based on the total mass of the preparation.

5. Preparation according to any of the preceding claims, **characterized in that** the weight ratio between antimicrobial and/or antiperspirant compounds and zinc salts is selected within the range from 4000:1 to 2:1.

6. Preparation according to any of the preceding claims, **characterized in that** the preparation is not a microemulsion.

7. Use of zinc salts of the carboxylic acids in combination with one or more antiperspirant compounds in cosmetic or dermatological preparations for increasing the deodorizing action of said preparations after topical application, **characterized in that** the zinc salt selected is zinc citrate, the antiperspirant compounds selected are aluminium salts and the preparations contain no active ingredients for inhibiting carrier proteins and/or inhibitors of the expression of the androgen receptor, more particularly no resveratrol, epigallocatechin-3-gallate, flufenamic acid, monensin, tunicamycin, amphmycin, diumycin, showdomycin, tsushimycin, amphotericins, mycospocidin, streptovirudin and/or D-glucosamines.

8. Use of zinc salts of the carboxylic acids in combination with one or more antiperspirant compounds in cosmetic or dermatological preparations for reducing or preventing body odour for up to 48 hours after topical application of the preparation, **characterized in that** the zinc salt selected is zinc citrate, the antiperspirant compounds selected are aluminium salts and the preparations contain no active ingredients for inhibiting carrier proteins and/or inhibitors of the expression of the androgen receptor, more particularly no resveratrol, epigallocatechin-3-gallate, flufenamic acid, monensin, tunicamycin, amphmycin, diumycin, showdomycin, tsushimycin, amphotericins, mycospocidin, streptovirudin and/or D-glucosamines.

## Revendications

1. Préparation cosmétique ou dermatologique comprenant un ou plusieurs sels de zinc d'acides carboxyliques et un ou plusieurs composés à activité antisudorale qui sont différents des sels de zinc, **caractérisée en ce qu'**en tant que sel de zinc on choisit le citrate de zinc, en tant que composés à activité antisudorale on choisit des sels d'ammonium et dans les préparations ne sont pas contenues de substances actives destinées à l'inhibition de protéines porteuses et/ou d'inhibiteurs de l'expression du récepteur d'androgène, en particulier pas de resvératrol, de 3-gallate d'épigallocatéchol, d'acide flufénamique, de monensine, de tunicamycine, d'amphmycine, de diumycine, de showdomycine, de tsushimycine, d'amphotéricines, de mycospocidine, de streptovirudine et/ou de D-glucosamines.

2. Préparation selon l'une quelconque des revendications précédentes, **caractérisée en ce qu'**en tant que composé à activité antisudorale on choisit des chlorhydrates d'aluminium.

3. Préparation selon l'une quelconque des revendications précédentes, **caractérisée en ce qu'**on choisit la proportion pondérale de sels de zinc dans la plage de 0,001 à 5 % en poids, en particulier 0,01 à 0,5 % en poids, par rapport à la masse totale de la préparation.

4. Préparation selon l'une quelconque des revendications précédentes, **caractérisée en ce qu'**on choisit la proportion pondérale de composés à activité antimicrobienne et/ou antisudorale dans la plage de 1 à 30 % en poids, par rapport à la masse totale de la préparation.

5. Préparation selon l'une quelconque des revendications précédentes, **caractérisée en ce qu'**on choisit le rapport pondéral entre composés à activité antimicrobienne et/ou antisudorale et sels de zinc dans la plage de 4 000 : 1 à 2 : 1.

6. Préparation selon l'une quelconque des revendications précédentes, **caractérisée en ce que** la préparation n'est pas une microémulsion.

7. Utilisation de sels de zinc des acides carboxyliques en association avec un ou plusieurs composés à activité antisudorale dans des préparations cosmétiques ou dermatologiques pour l'augmentation de l'effet déodorant de ces préparations après application topique, **caractérisée en ce qu'**en tant que sel de zinc on choisit le citrate de zinc, en tant que composés à activité antisudorale on choisit des sels d'ammonium et dans les préparations ne sont pas contenues de substances actives destinées à l'inhibition de protéines porteuses et/ou d'inhibiteurs de l'expression du récepteur d'androgène, en particulier pas de resvératrol, de 3-gallate d'épigallocatéchol, d'acide flufénamique, de monensine, de tunicamycine, d'amphmycine, de diumycine, de showdomycine, de tsushimycine, d'amphotéricines, de mycospocidine, de streptovirudine et/ou de D-glucosamines.

8. Utilisation de sels de zinc des acides carboxyliques en association avec un ou plusieurs composés à activité antisudorale dans des préparations cosmétiques ou dermatologiques pour la diminution ou l'empêchement de l'odeur corporelle pendant jusqu'à 48 heures après application topique de la préparation, **caractérisée en ce qu'**en tant que sel de zinc onc choisit le citrate de zinc, en tant que composés à activité antisudorale on choisit des sels d'ammonium et dans les préparations ne sont pas contenues de substances actives destinées à l'inhibition de protéines porteuses et/ou d'inhibiteurs de l'expression du récepteur d'androgène, en particulier pas de resvératrol, de 3-gallate d'épigallocatéchol, d'acide flufénamique, de monensine, de tunicamycine, d'amphmycine, de diumycine, de showdomycine, de tsushimycine, d'amphotéricines, de mycospocidine, de streptovirudine et/ou de D-glucosamines.
